# EUROPÄISCHE PATENTANMELDUNG

(11) **EP 1 369 693 A1**
(43) Veröffentlichungstag der Anmeldung: **10.12.2003**
(21) Anmeldenummer: 02012516.7
(22) Anmeldetag: 04.06.2002
(51) Int. Cl.: G01N 33/564, G01N 33/569, G01N 33/68

(54) **Verfahren zur Sepsisdiagnose und zur Kontrolle von Spenderblut durch Bestimmung von anti-Asialo-Gangliosid-Antikörpern**

(71) Anmelder: B.R.A.H.M.S Aktiengesellschaft, 16761 Hennigsdorf (DE)
(72) Erfinder: Bergmann, Andreas, Dr., 12351 Berlin (DE)
(74) Vertreter: Andrae, Steffen, Dr.

(57) **Zusammenfassung**

Verfahren zur Früherkennung und Erkennung, für die Verlaufsprognose und die Beurteilung des Schweregrads und zur therapiebegleitenden Verlaufsbeurteilung von Sepsis und sepsisähnlichen systemischen Infektionen sowie zur Abschätzung der Gefährdung von Sepsisrisikopatienten durch die Ausbildung einer Sepsis, bei dem man die Anwesenheit und/oder Menge von anti-Asialo-G_{M1}-Antikörpern (anti-AG_{M1}-Antikörpern) und damit kreuzreagierenden Antikörpern in einer biologischen Flüssigkeit eines Patienten oder Sepsisrisikopatienten bestimmt und aus deren Anwesenheit und/oder Menge Schlüsse hinsichtlich des Vorliegens, des zu erwartenden Verlaufs, des Schweregrads oder des Erfolgs einer Therapie der Entzündungserkrankung oder Sepsis oder hinsichtlich der Gefährung eines Sepsisrisikopatienten zieht.

Wird das Verfahren unter Einsatz von Spenderblut durchgeführt, ermöglicht es die Aussonderung potentiell schädlicher Blutkonserven.

## Beschreibung

Die vorliegende Erfindung betrifft ein neues Verfahren für die medizinische Diagnostik, insbesondere die präventive Diagnostik, von Sepsis und sepsisähnlichen systemischen Entzündungserkrankungen, und davon abgeleitet, auch ein Verfahren zur Kontrolle von Spenderblut, z.B. im Sinne eines Screenings von Blutbanken. Sie beruht auf dem erstmaligen Nachweis stark erhöhter Konzentrationen von anti-Gangliosid-Autoantikörpern, insbesondere von anti-Asialo-G_{M1}-Autoantikörpern und damit kreuzreagierenden Antikörpern, z.B. anti-GM₁-Antikörpern, vom IgG- und IgA-Typ, in den Seren von Sepsis-Patienten.

Insbesondere betrifft die vorliegende Erfindung ein Verfahren zur Früherkennung einer septischen Reaktion bei einem menschlichen Patienten (Sepsisrisikopatienten), bei dem aufgrund z.B. eines vorausgehenden medizinischen Eingriffs und/oder eines Traumas (Unfall, Verbrennung, Kriegsverletzungen, Dekubitus u.ä.) ein erhöhtes Risiko zur Entwicklung einer septischen Reaktion besteht, sowie zur Abschätzung der potentiellen Gefährdung eines Patienten durch eine septische Reaktion vor z.B. einem medizinischen Eingriff oder unmittelbar nach einem Trauma, wenn diese von einer solchen Art sind, dass sich in deren Folge als gefährliche Komplikation eine Sepsis entwickeln kann.

Das Verfahren hat dabei einen besonderen Wert als Risiko-Ausschlußverfahren, d.h für die Verneinung einer akuten Gefährdung duch Sepsis aufgrund eines negativen Nachweises der o.g. Antikörper.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien, Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α, Interleukin-1) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den der einschlägigen veröffentlichten Literatur entnehmbaren Kenntnissen über das Auftreten und die mögliche Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol. 33, 1999, 55-131; und C.Gabay, et al., "Acute Phase Proteins and Other Systemic Responses to Inflammation", The New England Journal of Medicine, Vol. 340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Eine eingeführte, als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol. 28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Ergebnisse dieser Anstrengungen finden sich in zahlreichen Patentanmeldungen der Anmelderin, und zwar in DE 198 47 690 A1 bzw. WO 00/22439 sowie in einer Reihe von noch unveröffentlichten deutschen (DE 101 19 804.3 bzw. PCT/EP02/04219; DE 101 31 922.3; DE 101 30 985.6) bzw. europäischen Patentanmeldungen (EP 01128848.7; EP 01128849.5; EP 01128850.3; EP 01128851.1; EP 01128852.9; EP 01129121.8; EP 02008840.7; und EP 02008841.5). Auf den Inhalt der genannten Patente und Patentanmeldungen wird hiermit durch ausdrückliche Bezugnahme zur Ergänzung der vorliegenden Beschreibung verwiesen.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol. 18, 2000, 917-918. Angesichts der bisherigen eher enttäuschenden Ergebnisse derartiger therapeutischer Ansätze besteht ein hohes Interesse daran, weitere möglichst entzündungs- bzw. sepsisspezifische endogene Biomoleküle zu identifizieren, die auch als therapeutische Targets neue Erfolgsaussichten für die Entzündungsbekämpfung eröffnen.

Bei allen oben bzw. in den genannten älteren Anmeldungen erwähnten Biomarkern handelt es sich physiologische Peptid- bzw. Proteinmoleküle, die z.B. Enzymcharakter oder den Charakter von (Pro-)Hormonen aufweisen oder definierte Zellbruchstücke sind. Proteine vom Immunglobulin-Typ, insbesondere vom IgG- und IgA-Typ, d.h. Antikörper, wurden bisher als diagnostisch relevante Biomarker für Sepsis, insbesondere eine bakteriell bedingte Sepsis, oder für eine besondere Gefährdungslage im Hinblick auf eine Sepsisentstehung bzw. bei einer fortschreitenden Sepsiserkrankung nicht diskutiert.

Es ist eine Grundaufgabe der vorliegenden Erfindung, einen weiteren Sepsisparameter zu finden, der im Rahmen der Sepsisdiagnostik und Sepsisverhütung bestimmt werden kann und ggf. die Einleitung von Maßnahmen zur Sepsisvorbeugung und Sepsisverhütung ermöglicht.

Diese Aufgabe wird durch ein Verfahren gemäß Anspruch 1 und dessen bevorzugte Ausführungsformen gemäß den Ansprüchen 2 bis 9 gelöst.

Von dem Verfahren der Ansprüche 1 bis 9 leiten sich weitere Verfahren zur Gesundheitsvorsorge ab, die sich als Verfahren zur Kontrolle von Spenderblut und von exogenen Substanzen bezeichnen lassen und in den Ansprüchen 10 und 12 und den darauf zurückbezogenen Ansprüchen 11 und 13 zusammengefaßt sind.

Weitere Ausführungsformen der vorliegenden Erfindung ergeben sich aus den nachfolgenden Erläuterungen und Beispielen.

Die vorliegende Erfindung beruht, allgemein gesprochen, auf der außerordentlich überraschenden Feststellung, dass ein bestimmter, an sich aus anderen Zusammenhängen bekannter Antikörper bzw. Autoantikörper, soweit derzeit experimentell überprüfbar war, in getesteten Seren von Sepsispatienten mit außerordentlicher Häufigkeit diagnostisch signifikant erhöht gefunden wird, während der gleiche Antikörper bei gesunden Normalpersonen nicht oder nur in deutlich geringeren Mengen nachweisbar ist. Die Serumproben von Sepsispatienten, in denen der Antikörper signifikant erhöht gefunden wurde, waren zu einem erheblichen Teil bereits kurz nach einem Sepsisrisiko-Ereignis (z.B. etwa 2h nach z.B. einer Operation, einem Unfall, einer Verbrennung) von Patienten gewonnen worden, die erst später die vollen Symptome einer Sepsis entwickelten. Das Auftreten von spezifischen Antikörpern mit einer hohen Sensitivität (richtiger Erkennung von Proben von Sepsispatienten) zu einem derartige frühen Zeitpunkt der Sepsisentwicklung war sehr überraschend und hat wichtige Konsequenzen im Hinblick auf die Entstehung derartiger Antikörper und die negative Rolle, die sie im Rahmen eines Sepsisgeschehens spielen bzw. spielen können. Darauf wird weiter unten noch genauer eingegangen werden.

Durch die erfindungsgemäße Bestimmung von anti-Asialo-G_{M1}-Antikörpern und damit kreuzreagierenden, d.h. an Asialo-G_{M1} bindenden, Gangliosidantikörpern wird es gemäß der vorliegenden Erfindung möglich, bei Sepsisrisikopatienten mit hoher Zuverlässigkeit eine Anfälligkeit für eine Sepsisentwicklung im Sinne einer erhöhten individuellen Gefährdungslage oder eine akute Gefährdung durch eine sich bereits entwickelnde Sepsis zu erkennen. Gleichzeitig werden bei einem negativen Ergebnis der Antikörperbestimung solche Patienten identifiziert, bei denen eine derartige erhöhte Gefährdung nicht besteht bzw. keine Sepsisentwicklung eingesetzt hat.

Soweit der bestimmte Antikörper auch bei einigen speziellen anderen Erkrankungen in erhöhten Konzentrationen auftritt, ist in der Regel eine korrekte Interpretation der Meßergebnisse für die Zwecke der Sepsisdiagnostik unter Berücksichtigung zusätzlicher klinischer Befunde und der Vorgeschichte eines Sepsisrisikopatienten ohne größere Schwierigkeiten möglich. Das gilt sowohl für die bekannten Neuropathien, bei denen anti-Gangliosid-Antikörper gefunden werden, als auch für Krebspatienten, bei denen derartige Antikörper ebenfalls im Normalfalle erhöht sind. Die Erhöhung von anti-Gangliosid-Antikörpern in den Seren Krebspatienten ist Gegenstand der etwas älteren, unveröffentlichten Patentanmeldung EP 02009884.4 der Anmelderin, worin nachgewiesen wird, dass die gleichen Antikörper, die im Rahmen des erfindungsgemäßen Verfahrens zur Sepsisdiagnose bestimmt werden, auch als universelle Biomarker für maligne neoplastische Erkrankungen nahezu beliebiger Art dienen können. Auf den gesamten Inhalt der genannten älteren Anmeldung und der dazu parallelen, therapeutische Aspekte betreffenden Patentanmeldung EP 02009882.8 wird, insbesondere was die allgemeinen Erläuterungen zu Gangliosiden und zur vorbekannten Rolle von anti-Gangliosid-Antikörpern angeht, zur Ergänzung der Ausführungen in der vorliegenden Anmeldung ausdrücklich verwiesen.

Wie weiter unten noch dargelegt werden wird, besteht ferner Grund zu der Annahme, dass die bei Sepsis - wie auch bei malignen Neoplasmen, vgl. EP 02009884.4 und EP 02009882.8 - signifikannt erhöht gefundenen anti-Asialo-G_{M1}-Antikörper und damit kreuzreagierenden Antikörper im Rahmen sowohl einer Sepsis als auch der Krebsentstehung durch eine negative Beeinflussung der normalen Immunreaktion eine möglicherweise entscheidende krankheitsfördernde Rolle spielen, und zwar im Sinne einer Zerstörung oder Hemmung/Desaktivierung der "Natürliche Killerzellen" (NK-Zellen) genannten cytotoxischen Lymphozyten, die ein wichtiges Glied der Immunantwort sind.

Es ist daher wichtig, einem Patienten derartige Antikörper nicht ungewollt von außen, z.B. mit der Verabreichung von Spenderblut, zuzuführen und ferner solche Faktoren aus dem menschlichen Umfeld zu ermitteln, die zu einer Sensibilisieurng im Hinblick auf die Bildung derartiger anti-Asialo-Antikörper in normalen Personen führen können. Daraus ergeben sich weitere, nachfolgend erläuterte Aspekte der vorliegenden Erfindung, nämlich einerseits die Bestimmung derartiger Antikörper zur Kontrolle von Blutkonserven bzw. in Spenderblut zum Zweck eines Blutkonserven-Screenings (Blutbanken-Screenings), andererseits die Testung von substanzgebundenen Umweltfaktoren auf ihre potentielle Fähigkeit, die Entstehung dieser Antikörper durch molekulare Mimikry hervorzurufen.

Die vorliegende Erfindung betrifft daher in weiteren Aspekten auch Verfahren zur Bestimmungen von anti-Asialo-G_{M1}-Antikörpern und damit kreuzreagierenden Antikörpern, bei denen diese nicht in Patientenseren zur Sepsis- oder Krebsdiagnose bestimmt werden, sondern zur Kontrolle von Spenderblut , z.B. in Blutkonserven, bestimmt werden, um die Immunreaktion eines Patienten, dem dieses Blut zugeführt wird, nicht zu schädigen, und zwar durch Inaktivierung seiner NK-Zellen und Störung ihrer Funktion. Eine solche Bestimmung unterscheidet sich nicht grundsätzlich von einer Bestimmung der gleichen Antikörper in einer Blutprobe (Serumprobe) eines Patienten, lediglich die Herkunft der Blutprobe und der Zweck der Durchführung der Bestimmung (Screening) sind andere.

Wie nachfolgend ebenfalls noch kurz erwähnt wird, ist bereits bekannt, dass in einem Menschen die Bildung von mit Gangliosiden wie G_{M1} reaktiven Antikörpern auch durch eine bakterielle Exposition (z.B. Infektionen mit *Campylobacter jejuni* oder auch *Helicobacter pylori)* ausgelöst werden kann (vgl. die entsprechenden Ausführungen in der älteren Anmeldung EP 02009884.4 und die darin genannte Literatur). Es ist daher davon auszugehen, dass weitere molekulare Strukturen existieren, die dem Kohlenhydrat-Teil von Gangliosiden ähneln und daher auf ähnliche Weise wie die genannten Bakterien durch molekulare Mimikry bei Menschen potentiell eine Bildung von anti-AG_{M1}-Antikörpern bzw. damit kreuzreagierenden Antikörpern veranlassen können, in der menschlichen Umwelt in verschiedenster Form vorkommen können. Es ist daher eine weitere Aufgabe, die sich aus den in dieser Anmeldung und in den genannten älteren Anmeldungen der Anmelderin beschriebenen diagnostischen Ergebnissen ableitet, ein Verfahrenzu schaffen, das es ermöglicht, Ganglioside, insbesondere Asialo-G_{M1}, vortäuschende Substanzen in der Umwelt als solche zu identifizieren und dadurch eine ansonsten möglicherweise nicht feststellbare Gefährlichkeit derartiger Substanzen für die menschliche Gesundheit zu ermitteln.

Diesem Zweck kann eine Screening-Verfahren dienen, bei dem man als derartige Substanzen in Betracht kommende Substanzen mit einem Assay-System aus anti-AG_{M1}-Antikörpern und spezifischen Bindern dafür, z.B. AG_{M1} in immobilisierter Form, in Kontakt bringt und den sich als Kompetition äußernden Einfluß der zu testenden Substanzen auf die Bindung der anti-AG_{M1}-Antikörper an ihren im Assay verwendeten spezifischen Binder ermittelt. In einen solchen Test können als Quelle für anti-AG_{M1}-Antikörper z.B. direkt Seren von Patienten, für die hohe Antikörper-Titer gemessen wurden, eingesetzt werden, und die relative kompetitive Beeinträchtigung der spezifischen Bindung an den spezifichen Binder kann auf im wesentlichen gleiche Weise bestimmt werden wie bei der Bestimmung der Antikörper in einem Serum oder in einer Blutprobe, nur dass der Reaktionsmischung zusätzlich eine zu testende Fremdsubstanz zugesetzt wird und das erhaltene Ergebnis mit einem Bezugswert für die substanzfreie Reaktionsmischung verglichen wird. Unter diesem Aspekt betrifft die vorliegenden Erfindung somit auch ein Verfahren, das als Verfahren zum Umweltscreening angesehen werden kann.

Nachfolgend werden die Auffindung des erfindungsgemäßen Verfahrens für die Sepsisdiagnose unter Präsentation der diesem Verfahren zugrundeliegenden Meßwerte sowie eine derzeit bevorzugte Art seiner praktischen Durchführung noch in näheren Einzelheiten erläutert.

Anschließend werden eine Deutung und ex-post-Plausibilisierung des erfindungsgemäßen Verfahrens im Lichte wissenschaftlicher Veröffentlichungen, die mit dem Gegenstand der vorliegenden Erfindung in einen Zusammenhang gebracht werden können, gegeben, die zeigen, dass die erfindungsgemäß bestimmten anti-Gangliosid-Antikörper bzw. Autoantikörper, insbesondere anti-AG_{M1}-Antikörper und damit kreuzreagierende Antikörper, für das Sepsisgeschehen, insbesondere für die Entstehung einer Sepsis und deren Verlauf, aufgrund ihres Einflusses auf die Funktion der NK-Zellen eine entscheidende Rolle spielen, woraus sich einerseits Hinweise auf neue therapeutische Ansätze zur Sepsisprävention, Sepsishemmung sowie ggf. Sepsistherapie ergeben und woraus sich die bereits erwähnten weiteren präventiven Aspekte der vorliegenden Erfindung ableiten lassen.

Die vorliegende Erfindung ist ein Ergebnis der intensiven Forschungen der Anmelderin auf dem Gebiet der klinischen Diagnose von Autoimmunerkrankungen und der Sepsis. Die Forschungen gingen im vorliegenden Fall aus von der bekannten Erkenntnis, dass zu den Antikörpern, die in der Literatur im Zusammenhang mit Autoimmunerkrankungen, insbesondere nervenschädigenden, neuropathischen Autoimmunerkrankungen, diskutiert werden, auch bestimmte Anti-Gangliosid-Antikörper gehören.

Ganglioside sind Glykolipide, die Bestandteile der extrazellulären Seite der Plasmamembran tierischer Zellen sind und als solche auch im Nervengewebe auftreten. Sie enthalten pro Mol mehrere Monosaccharid-Einheiten, jedoch keinen PhosphorGehalt und werden den Sphingolipiden zugeordnet. Verglichen mit Proteinen sind sie eher niedermolekulare Biomoleküle. Die Ganglioside, an die die im Rahmen der vorliegenden Erfindung diskutierten Antikörper binden, sind in erster Linie das in der vorliegenden Anmeldung als AG_{M1} abgekürzte Asialo-G_{M1} und das als G_{M1} abgekürzte zugehörige Monosialo-Gangliosid, für das die Anmelderin zeigen konnte, dass die in Seren gefunden Antikörperpopulationen wenigstens zu überwiegenden Teilen selektiv von beiden Gangliosiden (AG_{M1} und/oder G_{M1}) gebunden werden. G_{M1} ist ein Gangliosid, das eine Polysaccharidkette aus 4 Zucker-Monomereinheiten aufweist, die zwei D-Galactoseeinheiten, eine N-Acetylgalactosamin- und eine D-Glucoseeinheit umfassen, wobei letztere an einen sogenannten Ceramidteil gebunden ist. An die innerhalb der Polysaccharidkette angeordnete D-Galactoseeinheit ist bei dem Gangliosid G_{M1} ein N-Acetylneuraminsäurerest (NANA; Sialsäure- oder o-Sialinsäurerest; "Monosialo"-Rest) gebunden, der bei dem sialinsäurefreien Asialo-G_{M1} (AG_{M1}) fehlt.

Die genannten Ganglioside und verwandte Verbindungen werden mit zahlreichen wichtigen biologischen Funktionen des menschlichen Körpers in Zusammenhang gebracht, zu denen z.B. das axonale Wachstum und die neuronale Diffenzierung, Rezeptorenfunktionen und Beteiligungen an verschiedenen Immunreaktionen des Körpers sowie an der Signaltransduktion und Zell-Zell-Erkennung gehören. Nähere Einzelheiten finden sich z.B. in den in der Literaturliste zu der bereits genannten älteren Anmeldung EP 02009884.2 aufgeführten Veröffentlichungen.

Es ist seit langem bekannt, dass im menschlichen Körper Antikörper bzw. Autoantikörper auftreten können, die an die genannten und verwandte Ganglioside binden. Die physiologische Rolle derartiger Antikörper sowie ihre eventuelle Bedeutung für die klinische Diagnostik ist Gegenstand zahlreicher wissenschaftlicher Untersuchungen.

Der weitaus überwiegende Teil aller veröffentlichten Arbeiten befaßt sich mit der Rolle und der diagnostischen Signifikanz von Anti-Gangliosid-Antikörpern bei Neuropathien, z.B. bei immunvermittelten motorischen Neuropathien wie dem Guillain-Barré-Syndrom (Radikuloneuritis, Polyradikulitis) und dem verwandten (Miller-)Fisher-Syndrom. Auch im Zusammenhang mit der Alzheimer-Erkrankung wurde bereits von einem vermehrten Auftreten von Anti-G_{M1}-Autoantikörpern bei einigen Patienten berichtet. Ferner wurden sie auch bei einzelnen HIV-Patienten gefunden. Versuche ihrer Bestimmung im Zusammenhang mit bestimmten Krebsarten hatten nur wenig aussagekräftige, widersprüchliche Ergebnisse bzw. Ergebnisse mit einer niedrigen Sensitivität geliefert, bevor die Anmelderin eigene Untersuchungen durchführte, deren Ergebnisse in den genannten unveröffentlichten älteren Patentanmeldungen EP 02009884.4 und EP 02009882.1. niedergelegt sind.

Studiert man die einschlägigen wissenschaftlichen Veröffentlichungen zum Thema "Bestimmung von anti-Gangliosid-Antikörpern" genauer, fällt bei aller Ähnlichkeit vieler Beobachtungen auf, dass die Befunde und Aussagen bezüglich der zu beobachtenden Mengen - die in der Regel eher gering sind - und Typen der verschiedenen (Auto)-Antikörper bei den verschiedenen Patienten und Krankheitszuständen im einzelnen relativ stark voneinander abweichen. Das führte bereits zu dem Schluss, dass die Bestimmung derartiger Antikörper für die klinische Diagnostik nur von einem begrenzten bis zweifelhaften Wert sei (vgl. z.B. Michael Weller et al., Ganglioside antibodies: a lack of diagnostic specifity and clinical utility? J Neurol (1992) 239:455-459).

Die Anmelderin vermutete jedoch, dass der Grund für die z.T. erheblich divergierenden Literaturdaten im Methodologischen liegen könnte und dass möglicherweise aufgrund systematischer Fehler der angewandten Meßmethoden bisher noch keine wirklich zuverlässigen, aussagekräftigen und konsistenten Ergebnisse erhalten werden konnten. Die meisten der Bestimmungen, deren Ergebnisse veröffentlicht wurden, betrafen Patienten mit neurologischen Störungen und wurden mit Immunoassays vom ELISA-Typ durchgeführt, die so gestaltet waren, dass man mit einer Festphase arbeitete, an die - teilweise von den Autoren selbst aus biologischem Material gewonnene - Ganglioside gebunden waren. Diese Festphase wurde mit der flüssigen biologischen Probe umgesetzt, in der die zu bestimmenden Antikörper vermutet wurden. Nach der jeweils gewählten Inkubationszeit, einer Fest-Flüssig-Trennung und einem Waschen der Festphase wurden dann an diese gebundene humane Antikörper unspezifisch mit enzymmarkierten tierischen anti-human-Ig-Antikörpern markiert und bestimmt.

Ein Assay der genannten Art ist bei seiner Anwendung auf die Bestimmung von Anti-Gangliosid-Antikörpern außerordentlich anfällig für Störungen und Messfehler und kann nur bei sorgfältiger Standardisierung und Normierung zuverlässige, reproduzierbare Ergebnisse liefern. Eine der Ursachen dafür ist, dass die Qualität der Festphase, die durch Immobilisierung der relativ niedermolekulargewichtigen Ganglioside erhalten wird, anfällig für starke Schwankungen ist. Das ist teilweise dadurch begründet, dass vor der Umsetzung mit der flüssigen Probe restliche freie Bindungskapazitäten der Festphase abgesättigt werden müssen. Hierzu wird in der Regel Rinderserumalbumin, d.h. ein Protein verwendet. Dieser Schritt führt jedoch dazu, dass die unspezifische Bindung anderer Proteine, z.B. solcher vom IgG-Typ, aus der Probe sehr hoch wird, was zu einem starken Hintergrundsignal führt, vor dem die zu bestimmenden Antikörper bestimmt werden müssen. Ist jedoch die Empfindlichkeit eines Assays nicht sehr hoch - was bei Assays von ELISA-Typ in der Regel der Fall ist - können sich Hintergrundsignal und Messsignal so stark überlagern, dass unrichtige (falsch negative bzw. falsch positive) bzw. nicht zuverlässig reproduzierbare Messergebnisse erhalten werden.

Zu den verschiedenen angewandten Assaymethoden und den systematischen und praktischen Problemen bei der Anwendung derartiger Methoden auf die Bestimmung von anti-Gangliosid-Antikörpern kann z.B. verwiesen werden auf: Einar Bech et al., ELISA-Type Titertray Assay of IgM Anti-GM1 Autoantibodies, Clin.Chem. 40/7, 1331-1334 (1994); Alan Pestronk, MD et al., Multifocal motor neuropathy: Serum IgM anti-GM1 ganglioside antibodies in most patients detected using covalent linkage of GM1 to ELISA plates, Neurology 1997, 49:1289-1292; Mepur H. Ravindranath et al., Factors affecting the fine specifity and sensitivity of serum antiganglioside antibodies in ELISA, J.Immunol.Methods 169 (1994) 257-272; Armin Alaedini et al., Detection of anti-GM1 Ganglioside Antibodies in Patients with Neuropathy by a Novel Latex Agglutination Assay, J.Immunoassay, 21(4), 377-386 (2000); Armin Alaedini et al., Ganglioside Agglutination Immunoassay for Rapid Detection of Autoantibodies in Immune-Mediated Neuropathy, J.Clin.Lab.Anal. 15:96-99, 2001. Insbesondere Mepur H. Ravindranath et al., beschreiben in der genannten Veröffentlichung ausführlich einige der Grundprobleme der praktischen Anti-Gangliosid-Antikörperbestimmung.

Angesichts dieser Ausgangslage hatte sich die Anmelderin entschieden, das Problem der reproduzierbaren Bestimmung von anti-G_{M1}- bzw. anti-AG_{M1}-Antikörpern und ihrer diagnostischen Signifikanz z.B. bei Alzheimer-Patienten aufzugreifen und dabei die bei ihr als Produzentin von Assays für die klinische Diagnostik von Autoantikörpern vorhandenen besonderen Erfahrungen und Mittel zu nutzen. Für die interne Forschung entwickelte sie unter Einhaltung aller üblichen Qualitätsstandards Varianten einer verbesserten Modifikation der vorbekannten Antigangliosidassays. Die mit diesen verbesserten Assays durchgeführten Messungen von an G_{M1} bzw. AG_{M1} bindenden Antikörpern in Seren eines Vergleichskollektivs von Normalpersonen (Blutspendern) ohne relevante klinische Krankheitsbefunde sowie in Seren verschiedener Erkrankter lieferte, wie in den älteren Anmeldungen EP 02009884.4 und EP 02009882.8 beschrieben wird, einerseits das überraschende Ergebnis, dass in allen der Anmelderin zur Verfügung stehenden Seren von krebskranken Patienten gegenüber Normalpersonen hoch signifikant erhöhte Titer für anti-G_{M1}- bzw. anti-AG_{M1}-Antikörper vom IgA sowie vom IgG-Typ, dagegen nicht vom IgM-Typ, gefunden wurden. Andererseits lieferten diese Messungen das nicht weniger überraschende Ergebnis, dass Vergleichbares auch für die Anwesenheit der entsprechenden Antikörper in Seren von Sepsispatienten gilt. Die vorliegende Anmeldung beruht auf diesen letztgenannten Befunden und beschreibt technische Lehren, die sich daraus für die Sepsis-Prävention und -Therapie und die Gesundheitsvorsorge generell ergeben.

Obwohl die nachfolgend genauer beschriebenen Ergebnisse mit einem bestimmten verbesserten Ligandenbindungsassay ("Immunoassay") aus dem Labor der Anmelderin gewonnen wurden, ist die Nutzung der dabei gewonnenen Erkenntnisse nicht nur mit einem Assay des beschriebenen speziellen Formats möglich. Es wird vielmehr davon ausgegegangen, dass der nachfolgend beschriebene konkrete Assay für die in Frage stehende Antikörperbestimmung noch deutlich suboptimal ist, und dass kommerzielle Assays für die klinische Bestimmung von Anti-Gangliosid-Antikörpern, insbesondere von anti-AG_{M1}- und anti-G_{M1}-(Auto-)Antikörpern sich von dem beschriebenen Assay nach einer Optimierung in mehrfacher Hinsicht noch deutlich unterscheiden werden.

Die Verfahren zur Bestimmung der genannten Antikörper in einer biologischen Probe können beliebige bekannte Verfahren der Immundiagnostik sein, die zum selektiven Nachweis und zur Messung der Mengen von Antikörpern (Autoantikörpern) angewandt werden. Vorzugsweise werden die Antikörper mit Hilfe eines Ligandenbindungsassays bestimmt, bei dem man als Antigen zur Bindung der gesuchten Antikörper das jeweilige Gangliosid in immobilisierter Form verwendet. Zur Markierung der aus einer biologischen Probe spezifisch gebundenen Antikörper kann man dann auf irgendeine geeignete, an sich bekannte Weise markierte Anti-Human-Antikörper, markierte Gangliosidderivate oder deren Kohlenhydratstruktur simulierende Binder mit einer für das jeweilige Assayformat geeigneten Affinität verwenden.

Auch kompetitive Assayformate können besondere Vorteile bieten. Vorzugsweise wird dabei nicht mit einer Enzymmarkierung gearbeitet, sondern es wird eine andere Markierung gewählt, z.B. eine Markierung für eine Chemilumineszenz-Nachweisreaktion, z.B. ein Akridiniumester. Selbstverständlich ist vorzugsweise ein solcher Assay zur Antikörperbestimmung zu verwenden, der die benötigte hohe Sensitivität im Bereich der vorkommenden Antikörper-Konzentrationen gewährleistet und eine Separierung der Messignale vom Assay-Hintergrund ermöglicht.

Das Bestimmungsverfahren kann ferner an die Chip-Technologie angepaßt sein oder als Schnelltest (Point-of-Care-Test) ausgestaltet werden, wobei es auch möglich ist, die erfindungsgemäße Antikörperbestimmung im Rahmen einer Multiparameter-Bestimmung durchzuführen, bei der gleichzeitig mindestens ein weiterer Sepsis- oder Infektionsparameter bestimmt wird und bei der ein Messergebnis in Form eines Satzes von mindestens zwei Messgrößen gewonnen wird, der zur Feindiagnostik der Sepsis oder Infektion genauer ausgewertet wird. Als derartige weitere Parameter sind solche anzusehen, die aus der Gruppe der teilweise bekannten oder in den o.g. älteren Patentanmeldungen der Anmelderin offenbarten Parameter ausgewählt sind, d.h. der Gruppe, die insbesondere besteht aus Procalcitonin, CA 125, CA 19-9, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen, der Glycin-N-Acyltransferase (GNAT), der Carbamoylphosphat Synthetase 1 (CPS 1) und deren Fragmenten und dem C-reaktiven Protein (CRP) oder Fragmenten aller genannten Proteine.

Es kann dabei vorteilhaft sein, die Multiparameter-Bestimmung als Simultanbestimmung mittels einer Chiptechnologie-Messvorrichtung oder einer immunchromatographischen Messvorrichtung durchzufüren, bei der die Auswertung des mit der Messvorrichtung gewonnenen komplexen Messergebnisses mit Hilfe eines Computerprogramms erfolgt.

Zur Vermeidung von ungerechtfertigt engen und einschränkenden Auslegungen der in der vorliegenden Anmeldung und den zugehörigen Ansprüchen verwendeten Begriffe sollen nachfolgend einige der wichtigsten Begriffe für die Zwecke der vorliegenden Anmeldung besonders definiert werden:
- "Antikörper":: Dieser Begriff umfaßt, ohne zwischen verschiedenen Entstehungs- und Bildungsarten zu unterscheiden, Antikörper sowohl gegen externe Antigene als auch gegen körpereigene Strukturen, d.h. Autoantikörper, wobei letztere ggf. auch durch Antigen-Kreuzreaktionen aus Antikörpern gegen externe Antigene zu Autoantikörpern geworden sein können und ihre Bindungsfähigkeit gegen externe Antigene bewahrt haben können.
Wenn z.B. davon gesprochen wird, dass ein Antikörper "an Gangliosidstrukturen und an Gangliosidstrukturen simulierende Antigenstrukturen" bindet oder gegenüber "Gangliosiden bzw. bestimmten Gangliosiden reaktiv" ist, wobei reaktiv "im Sinne einer spezifischen Bindung reaktiv" bedeutet, soll er durch diese Definition hinreichend definiert sein, ohne dass z.B. seine spezifische Bindung auch an zusätzliche andere antigene Strukturen, oder seine praktische Bestimmung unter Verwendung von Reagenzien (zur Immobilisierung bzw. Markierung bzw. als Kompetitoren) mit molekularen Strukturen, die AG_{M1}, insbesondere dessen Kohlenhydratstuktur, nur simulieren, für die Definition als erfindungsgemäßer Antikörper eine Rolle spielen sollen.
- "kreuzreagierend": Wenn davon gesprochen wird, dass auch mit anti-Asialo-G_{M1}-Antikörpern kreuzreagierende Antikörper bestimmt werden sollen/können, so sind damit in erster Linie Antikörper gemeint, die im Sinne einer Kreuzreaktion auf eine vergleichbare Weise an Asialo-G_{M1}-Strukturen, wie sie als Determinanten auf NK-Zellen zu finden sind, binden und damit bezüglich dieser NK-Zellen vergleichbare physiologische Wirkungen wie anti-Asialo-G_{M1} -Antikörper aufweisen können.
- "Gangliosid": Im Rahmen der vorliegenden Erfindung steht der Begriff "Gangliosid" bei der Kennzeichnung des Bindungsverhaltens der zu bestimmenden Antikörper in erster Linie für das Ganglioside AG_{M1}. Der Begriff soll jedoch auch bisher noch nicht untersuchte verwandte Ganglioside erfassen, z.B. fukosylierte Ganglioside, wenn sich noch zeigt, dass auch an diese Ganglioside bindende Antikörper mit einer vergleichbaren diagnostischen Signifikanz in Sepsisseren gefunden werden.
- "Assay": Dieser Begriff umfaßt beliebige für eine Bestimmung der fraglichen (Auto-)Antikörper geeignete hoch sensitive Ligandenbindungsassays, ohne dass eine Beschränkung auf ein bestimmtes Assayformat (Sandwichassay, kompetitiver Assay, Agglutinationsassay) oder eine bestimmte Art der Markierung gewünscht wird. Es versteht sich, dass bestimmte Assayformate und/oder Markierungen anderen überlegen und daher bevorzugt sind (z.B. eine Chemilumineszenz- gegenüber einer Enzymmarkierung). Die Verwendung eines gegenüber dem nachfolgend konkret beschriebenen Assay verschlechterten oder verbesserten Assays soll jedoch nicht aus dem Bereich der Ansprüche herausführen, wenn er den in der vorliegenden Anmeldung definierten diagnostischen Zwecken dient.
- "Sensitivität": Eine hohe Sensitivität bedeutet im Rahmen der vorliegenden Erfindung, dass die Antikörper bei mindestens 50%, besser 70%, vorzugsweise mindestens 85% und nach stärker bevorzugt mindestens 95% aller Sepsispatienten gefunden werden.

Weitere Begriffsbedeutungen ergeben sich für den Fachmann aus der einleitenden und nachfolgenden Beschreibung der Erfindung und ihrer Ausführungsbeispiele.

In der nachfolgenden Beschreibung wird auf Figuren Bezug genommen, die zeigen:
- Fig. 1: eine graphische Darstellung der Ergebnisse der Messung von Antikörpern der IgG-Klasse, die an Monosialo-G_{M1} binden, in Seren von 137 Kontrollpersonen, gegenübergestellt den Ergebnissen der Vermessung von 89 Seren von Sepsispatienten;
- Fig. 2: die Ergebnisse einer Vermessung der gleichen Seren wie in Fig. 1 auf Antikörper der IgA-Klasse, die an Monosialo-G_{M1} binden;
- Fig. 3: die Ergebnisse der Bestimmung von Antikörpern der IgG-Klasse, die an Asialo-G_{M1} binden, in Seren von 30 Normalpersonen (Kontrollen), gegenübergestellt den Ergebnissen der Vermessung von 20 Seren von Sepsispatienten (alle Seren sind Teilkollektive der in den Figuren 1 und 2 vermessenen Seren);
- Fig. 4: die Ergebnisse der Bestimmung von Antikörpern der IgA-Klasse, die an Asialo-G_{M1} binden, in den gleichen Seren wie in Fig. 3;

### Antikörper-Bestimmungen:

### 1. Herstellung der Assaykomponenten:

### A. Herstellung von Teströhrchen (Coated Tubes; CT)

Es wurden drei Arten von Teströhrchen hergestellt: (a) Teströhrchen, an die die Ganglioside G_{M1} bzw. AG_{M1} gebunden waren, sowie (b) Teströhrchen mit einer BSA-Beschichtung zur Bestimmung des probenindividuellen Hintergrundsignals.
a) Zur Herstellung der gangliosidbeschichteten Teströhrchen (GA-CTs) wurden die Ganglioside (G_{M1} und AG_{M1}, jeweils bezogen von der Fa. Sigma, Deutschland) in Methanol gelöst und anschließend in PBS (phosphatgepufferte Salzlösung), pH 7,2, 25% Methanol, auf eine Konzentration von 5µg/ml verdünnt. Von dieser Lösung wurden jeweils 300 µl in Polystyrolröhrchen (Polystyrolröhrchen "Star" der Fa. Greiner, Deutschland) gegeben und bei Raumtemperatur für 16h inkubiert. Anschließend wurde der Inhalt der Röhrchen durch Absaugen entfernt, und die Röhrchen wurden zur Absättigung freier Bindungsstellen mit 4,5 ml 0,5% BSA (bovines Serumalbumin, proteasefrei, Fa. Sigma, Deutschland) in Wasser befüllt und 2h bei Raumtemperatur inkubiert. Dann wurde der Röhrcheninhalt dekantiert, und die Röhrchen wurden mit 0,2% Tween, 10 mM Tris/HCl, 10 mM NaCl, pH 7,5 befüllt und wieder dekantiert. Anschließend wurden die Röhrchen zur Antikörperbestimmung eingesetzt.
(b) Da Serumbestandteile an das zur Absättigung freier Bindungsstellen der Teströhrchenwand verwendete BSA binden, und der Grad einer solchen Bindung bei unterschiedlichen Seren sehr unterschiedlich sein kann, ist es erforderlich, für jedes Serum getrennt ein probenindividuelles Hintergrundsignal zu bestimmen.

Zu diesem Zweck wurden die gleichen Teströhrchen mit 4,5 ml 0,5% BSA in Wasser befüllt und 2h bei Raumtemperatur inkubiert. Dann wurde der Röhrcheninhalt dekantiert, und die Röhrchen wurden mit 0,2% Tween, 10 mM Tris/HCl, 10 mM NaCl, pH 7,5 befüllt und wieder dekantiert. Anschließend wurden die Röhrchen (HR-CTs) zur Bestimmung des probenindividuellen Hintergrundsignals eingesetzt.

### B. Herstellung von Akridiniumester-markierten anti human-IgG bzw. anti human-IgA Antikörpern (Tracer)

Ziegen anti human-IgG Antikörper (affinitätsgereinigt; grade II, Fa. Scantibodies, USA) bzw. Ziegen anti human-IgA Antikörper (affinitätsgereinigt; Fa. Sigma, Deutschland), jeweils 2 mg/ml in PBS, pH 7,4, 100 µl wurden mit je 10 µl Akridinium-NHS-ester (Fa. Hoechst, Deutschland; 1 mg/ml in Acetonitril; vgl. DE 36 28 573 A1) gemischt und für 20 min bei Raumtemperatur inkubiert. Nach Zusatz von 300 µl 20 mM Glycin, 50 mM NaCl, wurden die markierten Antikörper mittels Adsorptionschromatographie über Hydroxylapatit-HPLC gereinigt. Als Trennsäule wurde eine HPHT-Säule (120 mm x 8 mm) verwendet, äquilibriert in Lösemittel A (1 mM NaPO₄, pH 7,0, 10% Methanol, 0,1% Lubrol; "LM A"; Lubrol 17A17 wurde von der Fa. Serva, Deutschland bezogen). Die Durchflussgeschwindigkeit betrug 0,8 ml/min. Gebundene Antikörper wurden mittels eines linearen 40 min-Gradienten aus LM A/LM B (500 mM NaPO₄, pH 7,0, 10% Methanol, 0,1% Lubrol; "LM B") bei einer Flussgeschwindigkeit von 0,8 ml/min eluiert. Der Säulenausfluss wurde kontinuierlich auf UV-Absorption bei 280 nm (Protein) und 368 nm (Akridiniumester) vermessen. Nicht proteingebundener Akridiniumester wurden ungebunden aus der Säule eluiert und damit vollständig von den markierten Antikörpern abgetrennt. Die Antikörper wurden bei ca. 25 min eluiert. Nach der Bestimmung der Proteinkonzentration (BCA Methode) der HPLC-gereinigten markierten Antikörper erfolgte eine Verdünnung der Tracer auf eine Endkonzentration von 0,1 µg/ml in PBS, pH 7,2, 1 mg/ml Ziegen-IgG (Fa. Sigma, Deutschland) und 1% BSA.

### 2. Durchführung der Bestimmung von anti-Gangliosid Antikörpern

Zu untersuchende Proben (Humanseren) wurden 1:20 mit PBS, pH 7,2, 1 mg/ml Ziegen IgG, 1% BSA verdünnt. Hiervon wurden jeweils 10 µl in GA-CTs bzw. HR-CTs pipettiert. Anschließend erfolgte eine 16h Inkubation unter Schütteln (IKA-Schüttler KS250 basic, 400 U/min) bei 4°C.

Ungebundene Antikörper wurden durch 5maliges Befüllen/Dekantieren der Röhrchen mit 1 ml 0,2% Tween, 10mM Tris/HCl, 10 mM NaCl, pH 7,5 entfernt. Auf den Röhrchenoberflächen verbliebene Antikörper wurden durch Bindung markierter Ziegen anti human-IgG bzw. markierter Ziegen anti human-IgA nachgewiesen, indem man die Röhrchen mit jeweils 200 µl des jeweiligen Tracers (vgl. oben, 1.B.) und anschließend für 3h bei 4°C unter Schütteln inkubiert. Ungebundener Tracer wurde durch 5maliges Waschen (wie oben) entfernt.

Die Menge des auf der Röhrchenoberfläche verbliebenen markierten Antikörpers wurde mittels Lumineszenzmessung in einem Berthold LB.952T/16 Luminometer gemessen.

Das für GA-CTs erhaltene Lumineszenzsignal einer jeden Probe wurde dabei um das mit den HR-CTs gemessene jeweilige Hintergrundsignal für die gleiche Probe korrigiert. Das resultierende Signal (Differenzsignal) ist das Signal für an die Ganglioside G_{M1} bzw. AG_{M1} bindenden Antikörper aus der jeweiligen Probe. Als relative Kalibratoren für die Quantifizierung wurden Verdünnungsreihen von Proben mit einem hohen Gehalt an anti-Gangliosid-Antikörpern verwendet.

### 3. Vermessung von Seren von gesunden Normalpersonen (Kontrollen) und Sepsispatienten

Unter Verwendung der wie beschrieben hergestellten Teströhrchen und unter Anwendung des oben beschriebenen Verfahrens wurden die folgenden Reihenmessungen vorgenommen:

### Kontrollseren:

Als Kontrollseren für die Antikörper-Bestimmungen unter Verwendung von GA-CTs, die mit G_{M1} beschichtet waren, dienten 137 Kontrollseren (Blutspenderseren und - zur Vermeidung von Lebensalter-bedingten Einflüssen auf die Antikörperkonzentrationen - Seren von Normalpersonen aus Pflegeheimen verschiedenen Alters und von Mitarbeitern der Anmelderin). Für die Antikörper-Bestimmungen unter Verwendung von GA-CTs, die mit AG_{M1} beschichtet waren, wurde ein Teilkollektiv dieser Seren vermessen, das nur 30 Seren umfaßte.

### Testseren:

Als Testseren für die Antikörper-Bestimmungen unter Verwendung von GA-CTs, die mit G_{M1} beschichtet waren, dienten 89 Seren von Sepsispatienten. Für die Antikörper-Bestimmungen unter Verwendung von GA-CTs, die mit AG_{M1} beschichtet waren, dienten 20 Seren von Sepsispatienten (Teilkollektiv der o.g. 89 Seren). Zu jedem Testserum existierte eine klinische Dokumentation, die u.a. die Patienten-Vorgeschichte, den Zeitpunkt der Probennahme und den späteren Verlauf der Sepsiserkrankung betraf.

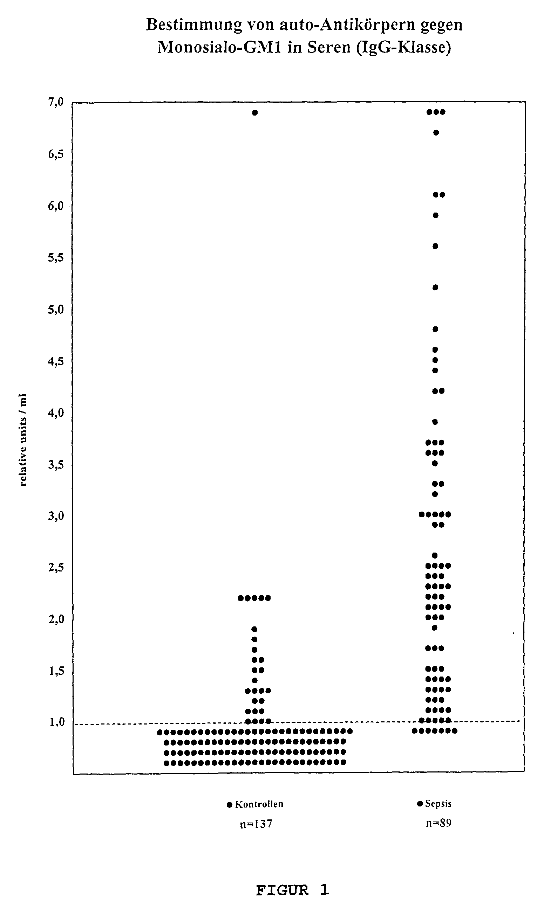

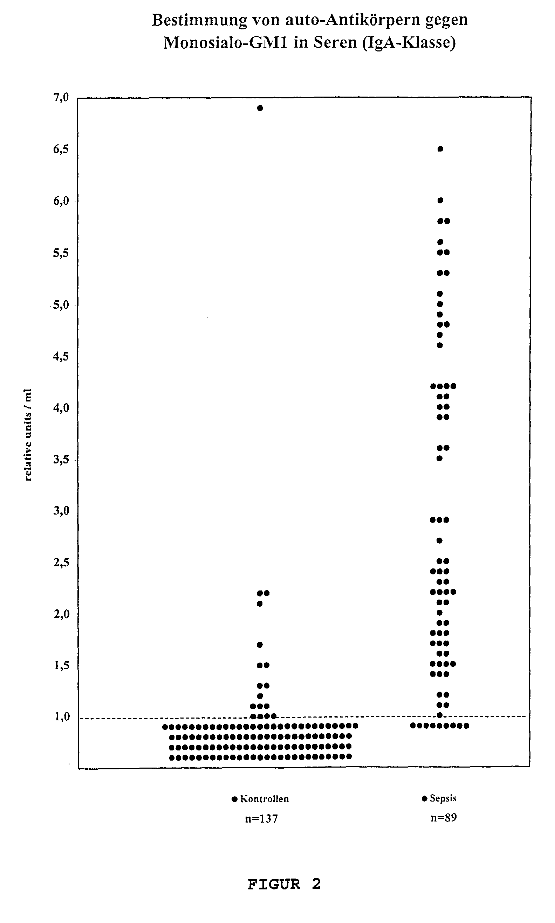

Die Ergebnisse der Bestimmungen von Antikörpern der Klassen IgG und IgA unter Verwendung von GA-CTs, die mit G_{M1} beschichtet waren, sind in den Figuren 1 und 2 gezeigt.

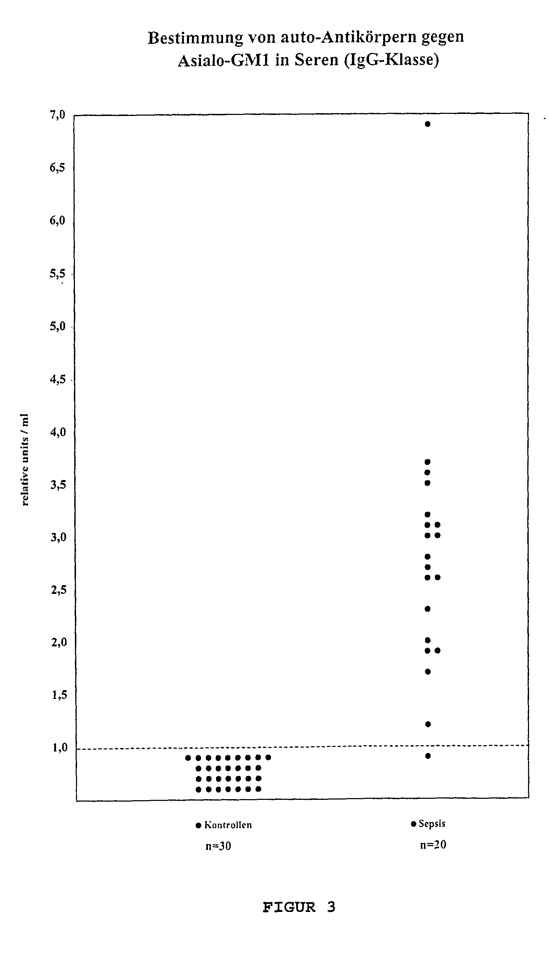

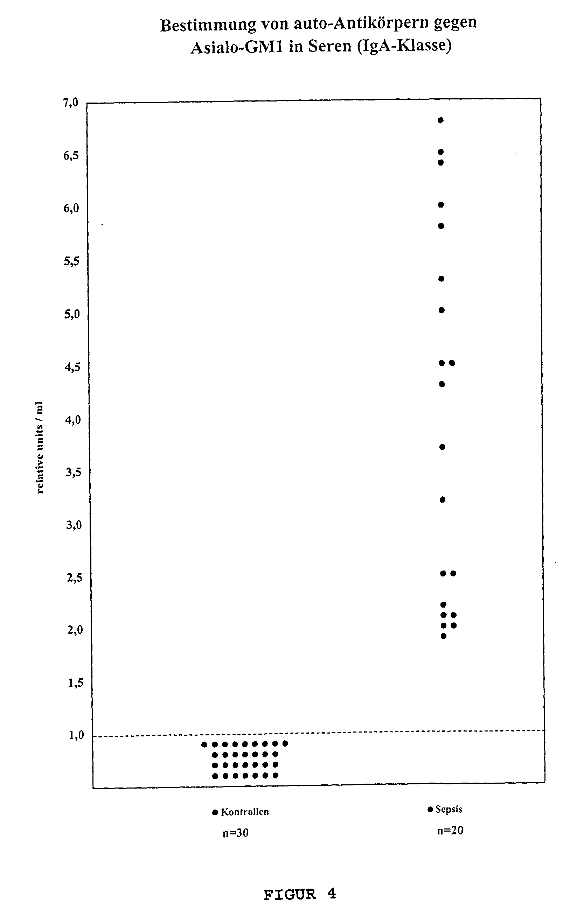

Die Ergebnisse der Bestimmungen von Antikörpern der Klassen IgG und IgA unter Verwendung von GA-CTs, die mit AG_{M1} beschichtet waren, sind in den Figuren 3 und 4 gezeigt.

### 4. Diskussion der Befunde der Bestimmung von anti-Gangliosid-Antikörpern in Kontrollseren und in Seren von Sepsispatienten

Wie die in den Figuren 1 bis 4 zusammengefassten Messergebnisse eindrücklich zeigen, ermöglicht die Bestimmung von Antikörpern der Klassen IgA und/oder IgG, die an Ganglioside (AG_{M1} und/oder G_{M1}) binden, ein klare Unterscheidung der Kontrollgruppe von den Sepsispatienten, indem in nahezu allen (82 von 89, d.h. 92%) der untersuchten Sepsisseren deutlich erhöhte AG_{M1} - bzw. G_{M1}-Antikörpertiter gefunden werden. Dabei scheint es, dass die Bestimmungen von IgA unter Verwendung von AG_{M1}-beschichteten Teströhrchen Meßergebnisse mit der höchsten Sensitivität (alle Sepsispatienten positiv) und Selektivität (keine Erfassung von Nicht-Sepsispatienten als positiv) liefern (wobei allerdings einschränkend zu berücksichtigen ist, dass aus praktischen Gründen die Zahl von nur 20 Bestimmungen geringer war als im Falle der 89 Bestimmungen unter Verwendung von G_{M1}-beschichteten Röhrchen).

Es ist ferner von Bedeutung, dass bei einem Versuch, analog zu den Bestimmungen von Antikörpern vom IgG- und IgA-Typ auch entsprechende Antikörper vom IgM-Typ zu bestimmen, in den Sepsisseren keine diagnostisch relevant erhöhten Spiegel für Antikörper vom IgM-Typ gefunden wurden (Ergebnisse nicht gezeigt).

Der Nachweis von deutlich erhöhten Konzentrationen von Antikörpern vom IgA und IgG-Typ auch in Patienten-Serumproben, die nur kurze Zeit (etwa 2h) nach dem "Sepsisrisiko-Ereignis" (z.B. Operation, Unfall, Verbrennung) gewonnen worden waren, sowie der mangelnde Nachweis von Antikörpern vom IgM-Typ, schließen es aus, dass die nachgewiesenen Antikörper erst infolge des "Sepsisrisiko-Ereignisses" bzw. einer damit verbundenen bakteriellen Infektion gebildet wurden. Das bedeutet aber, dass die Antikörper bei dem jeweiligen Sepsispatienten entweder schon vorher vorhanden waren und/oder dass die durch das Sepsis-Risikoereignis ausgelöst Aktivierung des vorsensibilisierten Immunsystems des Patienten in der Art eines "Booster"-Effekts eine intensive Antikörperproduktion ausgelöst hat.

Die Tatsache, dass die AG_{M1}- bzw. G_{M1}-Antikörper in im wesentlichen allen vermessenen Sepsisseren (92%) deutlich erhöht gefunden wurden, ist daher so zu interpretieren, dass die Ausbildung einer Sepsis entweder ursächlich mit dem vorherigen Vorhandensein der fraglichen Antikörper bei dem jeweiligen Patienten verknüpft ist oder wenigstens die Folge einer Aktivierung der aufgrund einer vorhergehenden Immunisierung bei dem Patienten bereits vorhandenen "molekularen Maschinerie" (in Form von B-Zellen) ist, die unter dem Einfluß des "Sepsisrisiko-Ereignisses" bzw. einer damit verbundenen Infektion ihre intensive Antikörperproduktion aufnimmt. Patienten ohne diese Antikörper bzw. die zu ihrer raschen Erzeugung erforderlichen Vorsensibilisierung entwickeln nach Maßgabe der bisherigen experimentellen Befunde eine Sepsis wahrscheinlich gar nicht oder nur erschwert.

Die beobachteten Befunde können plausibel gemacht werden: Es ist bekannt, dass die sogenannten "natürlichen Killerzellen" (NK-Zellen; cytotoxisch aktive Lymphozyten) an ihrer Oberfläche Asialo-G_{M1}-Strukturen aufweisen, an die anti-AG_{M1}-Antikörper spezifisch binden können und dadurch die NK-Zellen desaktivieren. So kann darauf verwiesen werden, dass es auf dem Gebiet von Tierexperimenten, bei den mit Versuchstieren gearbeitet wird, bei denen künstlich Tumoren erzeugt werden sollen, üblich ist, durch Gabe von anti-AG_{M1}-Antikörpern in Verbindung mit einem krebsauslösenden Karzinogen oder einem Tumorkeim die Immunabwehr des Versuchstiers auszuschalten, so dass sich der - im Tiermodell gewünschte - experimentelle Krebs entwickeln kann (Hugh F. Pross et al., Role of Natural Killer Cells in Cancer, Nat Immun 1993; 12:279-292; Lewis L. Lanier et al., Arousal and inhibition of human NK Cells, Immunological Reviews 1997, Vol. 155:145-154; Yoichi Fuji et al., IgG Antibodies to AsialoGM1 Are More Sensitive than IgM Antibodies to Kill *in vivo* Natural Killer Cells and Prematured Cytotoxic T Lymphocytes of Mouse Spleen, Microbiol.Immunol. Vol. 34(6), 533-542, 1990; N.Saijo et al., Analysis of Metastatic Spread and Growth of Tumor Cells in Mice with Depressed Natural Killer Activity by Anti-asialo GM1 Antibody or Anticancer Agents, J Cancer Res Clin Oncol (1984) 107: 157-163; Sonoku HABU et al., Role of Natural Kiler Cells against Tumor growth in Nude Mice - A Brief Review, Tokai J Exp Clin Med., Vol.8, No.5, 6: 465-468, 1983; Lewis L. Lanier, NK Cell Receptors, Annu. Rev. Immunol. 1998, 16: 359-93; Theresa L. Whiteside et al., The role of natural killer cells in immune surveillance of cancer; Current Opinion in Immunology 1995, 7:704-710; Tuomo Timonen et al., Natural killer cell-target cell interactions, Current Opinion in Cell Biology 1997, 9:667-673).

Aktive NK-Zellen spielen jedoch eine außerordentlich wichtige Rolle im Rahmen der Immunabwehr des Menschen, auch bei Sepsis bzw. schweren bakteriellen Infektionen. So beschreiben z.B. Shuiui Seki et al., in: Role of Liver NK Cells and Peritoneal Macrophages in Gamma Interferon and Interleukin-10 Production in Experimental Bacterial Peritonitis in Mice, Infection and Immunity, Vol. 66, No. 11, 1998, 5286-5294; die wichtige Rolle von NK-Zellen für die Produktion von entzündungsfördernden und entzündungshemmenden Cytokinen. Sie zeigen, dass eine künstliche Ausschaltung der NK-Zellen unter experimentellem Einsatz von anti-AG_{M1}-Antikörpern zu einer Inbierung der Produktion des entzündungshemmenden Interferon-γ führt. Auch eine Auswirkungen von chirurgischem Stress und einer Endotoxin-induzierten Sepsis auf die NK-Zell-Aktivität wurde bereits beschrieben, und zwar in: P.Toft et al., in: The effect of surgical stress and endotoxin-induced sepsis on the NK-cell activity, distribution and pulmonary clearance of YAC-1 and melanoma cells, APMIS 1999; 107:359-364. Ein möglicher Einfluss von physiologisch gebildeten Antikörpern mit NK-Zell-Reaktivität wird in keiner der genannten Arbeiten berücksichtigt.

Der Nachweis von natürlich vorkommenden anti-AG_{M1}-Antikörpern und damit kreuzreagierenden anti-Gangliosid-Antikörpern, z.B. anti-G_{M1}-Antikörpern, und die erhöhten Spiegel derartiger Antikörper in Seren von Sepsispatienten, bedeutet jedoch, dass derartige Antikörper eine bisher unberücksichtigte Einflußgröße auf die infektions- bzw. entzündungsspezifische Cytokinkaskade darstellen, indem sie in den natürlichen Cytokin-Regelkreis eingreifen und diesen durch Störung oder Ausschaltung der NK-Zellen veranlassen können, zu entgleisen und bei dem Patienten zu einer septischen Reaktion auszulösen.

Da, wie bereits erläutert, die in Sepsisseren gefundenen anti-AG_{M1}-Antikörper aufgrund ihrer Art und des Zeitpunkts ihres Auftretens nicht durch das akute sepsisauslösende Ereignis oder eine damit verbundene Neuinfektion entstanden sein können, sondern wenigstens als Anlage bereits vorhanden gewesen sein müssen, eignet sich die Bestimmung derartiger Antikörper auch zur Ermittlung der Gefährdungslage und für die Prognose bei einem Patienten, der als Sepsisrisikopatient einzustufen ist.

Die gemessene hohe Sensitivität macht die Bestimmung von anti-AG_{M1}-Antikörpern und damit kreuzreagierenden anti-Gangliosid-Antikörpern, insbesondere von solchen der IgGund/oder IgA-Klassen, daher zu einem vielversprechenden Bestimmungsverfahren für die Sepsisdiagnose, insbesondere für die Früherkennung und, wie oben erläutert wurde, auch für die Ermittlung der persönlichen Gefährdungslage eines Sepsisrisikopatienten bzw. die Prognose einer Sepsis.

Der wissenschaftlichen Literatur lassen sich Erkenntnisse, die das erfindungsgemäße Verfahren nahelegen könnten, nicht entnehmen. Es wurden nur einige wenige Arbeiten bekannt, bei denen anti-Gangliosid-Antikörper im Zusammenhang mit schweren akuten Infektionskrankheiten bestimmt wurden. Ein derartiger Fall ist die durch den Parasiten *Trypanosoma cruzi* hervorgerufene Chagas-Krankheit (vgl. D.H.Bronia et al., in: Ganglioside treatment of acute *Trypanosoma cruzi* infection in mice promotes long-term survival and parasitological cure, Annals of Tropical Medicine & Parasitology, Vol. 93, No.4, 341-350 (1999) und die darin zitierte Literatur). In der letztgenannten Arbeit wird spekuliert, dass eine beobachtete, deutlich vorteilhafte Wirkung einer Verabreichung von exogenen Gangliosiden an mit dem Parasiten *T. cruzi* infizierte Mäuse bei diesen die Produktion von anti-Gangliosid-Antikörpern auslösen könnte, die mit Glycolipiden der Hülle von *T. cruzi* reagieren und dadurch den Tod des Parasiten bewirken. Aufgrund der Befunde in der vorliegenden Anmeldung ist eine solche Erklärung wenig wahrscheinlich: Die im Falle der Chagas-Krankheit beobachteten anti-Gangliosid-Antikörper sind aufgrund ihrer die NK-Zellen hemmenden Wirkung kein heilungsfördernder, sondern ein krankheitsauslösender bzw. -fördernder Faktor. Durch die Verabreichung von exogenen, kaum antigenen Gangliosiden werden die anti-AG_{M1}-Antikörper nämlich nicht gebildet sondern wahrscheinlich blockiert, wodurch bei den Mäusen (bzw. bei Patienten) die Wirkung der NK-Zellen wiederhergestellt wird und das Immunsystem der Parasiten Herr werden kann. Die bekannten Arbeiten deuten auf keinerlei Zusammenhang von anti-Asialo-G_{M1}-Antikörpern mit der Entstehung und Verschärfung einer Sepsis hin und können daher das erfindungsgemäße Verfahren weder vorwegnehmen noch nahelegen.

Die Deutung der Befunde, die Grundlage für die vorliegende Erfindung sind, läßt sich noch wie folgt vertiefen: Die Sensibilisierung eines Patienten im Hinblick auf die Produktion von anti-Gangliosid-Antikörpern in Reaktion auf einen antigegen Reiz kann durch eine Allerweltsinfektion oder ggf. auch entsprechende Umweltsubstanzen erfolgt sein und danach latent lange vorhanden bleiben. Ist jedoch bei einem menschlichen Individuum, z.B. aufgrund einer bakteriellen Exposition (z.B. Infektionen mit *Campylobacter jejuni* oder auch *Helicobacter pylori),* die Produktion von anti-Asialo-G_{M1}-Antikörpern einmal initiiert bzw. stark erhöht, sind bei diesem Patienten die Voraussetzungen gegeben, dass in bestimmten physiologischen Stress-Situationen mit hoher NK-Zell-Aktivität (z.B. Zellentartung durch mutagene Ereignisse; eine Sepsis-Risikosituation) eine Schädigung der NK-Zellen und damit der Immunabwehr erfolgt, mit der Folge eines erhöhten Risikos, dass eine Abwehrreaktion, die durch z.B. einen "Sepsisrisiko-Stress" ausgelöst wird und ein Eingreifen der NK-Zellen erfordert, auch die Produktion der o.g. Antikörper stimuliert, und dass diese dann die Wirkung der NK-Zellen aufheben. Der Regelkreis der Immunantwort ist dann entscheidend gestört, und es kann sich eine Sepsis entwicklen.

Es ist also eher davon auszugehen, dass die in allen Sepsisseren, die im Rahmen der o.g. Bestimmungen vermessen wurden, signifikant erhöht gefundenen anti-AG_{M1}-Antikörpertiter eine der Voraussetzungen des Entstehens einer Sepsis sind und sich die Anwesenheit derartiger Antikörper negaitv auswirkt.

Da die mit Gangliosiden kreuzreagierenden Antikörper und die für deren Produktion erforderliche Prägung des Immunsystems bereits vor der Entwicklung einer Sepsis vorhanden sein müssen, kann die Bestimmung von anti-AG_{M1}-Antikörpern mit Vorteil erfindungsgemäß auch im Sinne der Ermittlung einer Disposition, d.h. als Bestimmung eines Sepsis-Risiko-Markers, erfolgen. Es kann in diesem Zusammenhang vorteilhaft sein, eine solche Bestimmung nach einer *in vivo* Stimulierung der Antikörperbildung eines Sepsisrisikko-Patienten, z.B. vor einer Operation, unter Verwendung unbedenklicher Stimulantien zu vorzunehmen. Angesichts der deutlich erhöht gefundenen IgA-Antikörper (vgl. Figuren 2 und 4) soll die Antikörperbestimmung ausdrücklich auch mit geeigneten Assays in Körpersekreten (z.B. Speichel, Schleim) erfolgen können.

Aus den obigen Ausführungen ergibt sich auch die Wichtigkeit einer Vermeidung einer externen Zufuhr von anti-AG_{M1}-Antikörpern, z.B. mit Spenderblut, an einen Patienten, insbesondere in einer Situation, in der an die Funtion seines Immmunsystems hohe Anforderungen gestellt werden, sowie der möglichst weitgehenden Vermeidung einer Exposition von Personen generell bezüglich antigener Substanzen, die Gangliosidstrukturen simulieren und dadurch zur Bildung von anti-Gangliosid-Antikörpern oder mit Gangliosidstrukturen kreuzragierenden Antikörpern führen können.

Die sich aufgrund er in dieser Anmeldung geschilderten Befunde ergebenden Konsequenzen für neuartige Verfahren zur Prävention, Hemmung und Therapie von septischen krankheitszuständen und zur allgemeinen Gesundheitsvorsorge sind Gegenstand einer gleichzeitig mit der vorliegenden Anmeldung eingereichten eigenen parallelen Patentanmeldung.

## Patentansprüche

1. Verfahren zur Früherkennung und Erkennung, für die Verlaufsprognose und die Beurteilung des Schweregrads und zur therapiebegleitenden Verlaufsbeurteilung von Sepsis und sepsisähnlichen systemischen Infektionen sowie zur Abschätzung der Gefährdung eines Sepsisrisikopatienten durch die Ausbildung einer Sepsis, **dadurch gekennzeichnet, dass** man die Anwesenheit und/oder Menge von anti-Asialo-G_{M1}-Antikörpern (anti-AG_{M1}-Antikörpern) und damit kreuzreagierenden Antikörpern in einer biologischen Flüssigkeit eines Patienten oder Sepsisrisikopatienten bestimmt und aus deren Anwesenheit und/oder Menge Schlüsse hinsichtlich des Vorliegens, des zu erwartenden Verlaufs, des Schweregrads oder des Erfolgs einer Therapie der Entzündungserkrankung oder Sepsis oder hinsichtlich der Gefährung eines Sepsisrisikopatienten zieht.

2. Verfahren nach Anspruch 1, **dadurch gekennzeichnet, dass** man anti-AG_{M1}- und/oder anti-G_{M1}-(Auto-)Antikörper vom IgGund/oder IgA-Typ bestimmt.

3. Verfahren nach Anspruch 1 oder 2, **dadurch gekennzeichnet, dass** die biologische Flüssigkeit Blut, eine Blutfraktion oder ein Sekret ist.

4. Verfahren nach einem der Ansprüche 1 bis 3, **dadurch gekennzeichnet, dass** man die Bestimmung mit Hilfe eines Ligandenbindungsassays vom Sandwichtyp oder vom kompetitiven Typ oder eines Agglutinationsassays durchführt.

5. Verfahren nach einem der Ansprüche 1 bis 4, **dadurch gekennzeichnet, dass** man die Bestimmung der Antikörper in einer Blutprobe eines Sepsisrisikopatienten nach einer vorherigen in vivo und/oder in vitro Stimulierung der Antikörperproduktion vornimmt.

6. Verfahren nach einem der Ansprüche 1 bis 5, **dadurch gekennzeichnet, dass** es im Rahmen einer Multiparameter-Bestimmung durchgeführt wird, bei der gleichzeitig mindestens ein weiterer Entzündungs- oder Infektionsparameter bestimmt wird und bei der ein Messergebnis in Form eines Satzes von mindestens zwei Messgrößen gewonnen wird, der zur Feindiagnostik der Sepsis ausgewertet wird.

7. Verfahren nach Anspruch 6, **dadurch gekennzeichnet, dass** im Rahmen der Multiparameter-Bestimmung neben den anti-Gangliosid-Autoantikörpern wenigstens ein weiterer Parameter bestimmt wird, der ausgewählt ist aus der Gruppe, die besteht aus den Proteinen Procalcitonin, CA 125, CA 19-9, S100B, S100A-Proteinen, LASP-1, löslichen Cytokeratin-Fragmenten, insbesondere CYFRA 21, TPS und/oder löslichen Cytokeratin-1-Fragmenten (SCY1F), den Peptiden Inflammin und CHP, Peptid-Prohormonen, der Glycin-N-Acyltransferase (GNAT), der Carbamoylphosphat Synthetase 1 (CPS 1) und dem C-reaktiven Protein (CRP) oder Fragmenten davon.

8. Verfahren nach Anspruch 6 oder 7, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

9. Verfahren nach Anspruch 8, **dadurch gekennzeichnet, dass** die Auswertung des mit der Messvorrichtung gewonnenen komplexen Messergebnisses mit Hilfe eines Computerprogramms erfolgt.

10. , Verfahren zur Qualitätskontrolle von Spenderblut für medizinische Zwecke, bei dem man in einer Probe des Spenderbluts die Anwesenheit und/oder Menge von anti-Asialo-G_{M1}-Antikörpern (anti-AG_{M1}-Antikörpern) und damit kreuzreagierenden Antikörpern, insbesondere anti-G_{M1}-Antikörpern, bestimmt und man im Falle eines positiven Nachweises derartiger Antikörper
- das Spenderblut aussondert oder
- einer Affinitätsreinigung zur Entfernung der bestimmten Antikörper unterzieht und erst nach einer anschließenden weiteren Antikörperbestimmung mit negativem Ergebnis an einen Patienten verabreicht.

11. Verfahren nach Anspruch 10, bei dem man als Spenderblut Blutkonserven aus einer Blutbank oder frisch gewonnenes Spenderblut untersucht.

12. Verfahren zur Auffindung und zum Nachweis von Einzelsubstanzen oder von Bestandteilen von Substanzgemischen, die Struktureigenschaften aufweisen, die Gangliosidstrukturen simulieren, bei dem man zu untersuchende Einzelsubstanzen oder Substanzgemische in einem Assaysystem prüft, das auf der Bindung von anti-Gangliosid-Antikörpern an einen spezifischen Binder und dem Nachweis der gebundenen Antikörper beruht, wobei man eine kompetitive Verminderung der Antikörperbindung an den spezifischen Binder in Anwesenheit der zu untersuchenden Substanz als Hinweis auf
- antikörperblockierende Eigenschaften der Substanz oder
- ein potentielles Gefährdungspotential der Substanz aufgrund einer Antigenwirkung unter Initiierung der Produktion von anti-AG_{M1}-Antikörpern oder damit kreureagierenden Antikörpern in Menschen ansieht.

13. Verfahren nach Anspruch 12, bei dem Einzelsubstanzen oder Substanzgemische geprüft werden, die der menschlichen oder tierischen Ernährung dienen und/oder aus medizinischen oder kosmetischen Gründen an Menschen verabreicht werden.
